(19) Europäisches Patentamt
European Patent Office
Office européen des brevets

(11) Publication number: **0 155 594**
**A2**

# (12) EUROPEAN PATENT APPLICATION

(21) Application number: 85102536.1

(22) Date of filing: 06.03.85

(51) Int. Cl.[4]: C 12 N 15/00
C 12 N 1/20
//(C12N1/20, C12R1:15),
C12R1:13, C12R1:125

(30) Priority: 06.03.84 US 586587

(43) Date of publication of application:
25.09.85 Bulletin 85/39

(84) Designated Contracting States:
DE FR GB IT

(72) Inventor: Walker, Graham
16 Lakeview
Arlington Mass. 02174(US)

(72) Inventor: Rao, Eswara
2202 Broughton Drive
Beverly Mass. 01915(US)

(72) Inventor: Shanabruch, William
20R Auburn Avenue
Somerville Mass. 02145(US)

(71) Applicant: MASSACHUSETTS INSTITUTE OF TECHNOLOGY
77 Massachusetts Avenue
Cambridge, MA 02139(US)

(72) Inventor: Sinskey, Anthony
285 Commonwealth Avenue
Boston Mass. 02116(US)

(72) Inventor: Yoshihama, Makato
Saitama 350-1
Sayama(JP)

(72) Inventor: Akedo, Makatsu
18 Pine Street
Boston Mass. 02111(US)

(72) Inventor: Follettie, Maximillian
474 Memorial Drive
Cambridge Mass.02139(US)

(74) Representative: Patentanwälte Zellentin
Zweibrückenstrasse 15
D-8000 München 2(DE)

## (54) Cloned plasmids of corynebacterium.

(57) A chimeric plasmid that serves as a shuttle vector for at least two species of bacteria is transformed into the bacteria cells while incubating the cells in the presence of glycine and in the absence of a chelating agent. A representative suitable plasmid is formed from the DNA of Corynebacterium and Escherichia bacteria.

EP 0 155 594 A2

March 6,1985
Eu 85 397 Gr/fr

# Cloned Plasmids Of Corynebacterium

This invention relates to the formation of DNA shuttle vectors capable of being cloned directly in Corynebacterium organisms and capable of beeing expressed in both Corynebacterium organisms and Escherichia organisms.

Preparation of protoplasts is well known, especially in gram-positive microorganisms. The technology for protoplast formation in both gram-positive and gram-negative microorganisms is discussed extensively by Hopwood, "Genetic Studies with Bacteria Protoplasts," Ann. Rev. Microbiol. 1981. 35, 237-72. The preponderance of techniques discussed by Hopwood relate to protoplast formation in Streptomyces and Bacillus organisms. The transformation of protoplasts is described at pages 263-268. Protoplast fusion techniques are reviewed by Peberdy, Enzyme Microb-Technol. 1980, vol.2, pages 23-29. Protoplast fusion techniques for obtaining genetic recombination in Brevibacterium flavum are discussed by Kaneko et al. in Agric. Biol. Chem., 43 (5), 1007-1013 (1979). Protoplast fusion in Corynebacterium organisms as described by Katsumata et al. involves the use of penicillin and lysozyme. Canadian Patent 1,105,859 also describes protoplast fusion as applied to Streptomyces.

Preparation of "co-integrate" plasmids for transformation of Streptomyces and Escherichia coli is described in U.S. Patent 4,273,875 and 4,332,900. EPO application 058,889 (Katsumata and Furuya) describes plasmid cloning vectors for Corynebacterium.

Several patents and patent applications concerned with strain development with species of Corynebacterium and Brevibacterium have appeared recently. For example:
A Method for Gene Expression; Kyowa Hakko Kogyo Co., Japan Kokai Tokkyo Koho No. 83,126,789.
Composite Plasmids, Ajinomoto Co., Inc., Japan, Eur.Pat.Appl.No. 93611.
Fusion of Microbial Protoplasts, Ajinomoto Co., Japan, Japan Kokai Tokkyo Koho No. 83,158,186.
Method for Fusion of Microbial Protoplasts, Ajinomoto Co., Japan Kokai Tokkyo Koho, No. 83,158, 134.
In addition, U.S. patent application Serial Number 489,289 entitled "Cloning Systems for Corynebacterium", filed April 28,1983, describes the production of shuttle vectors capable of being expressed in Corynebacterium organisms and Bacillus organisms.

A preliminary communication on the construction of a coryneform - Escherichia coli shuttle vector was reported by W.G. Shanbruch, G. C. Walker and A.J. Sinskey at the 1983 American Society of Microbiology meeting held the week of March 6th in New Orleans, LA.

Several of the coryneform bacteria belonging to the genera Corynebacteria, Brevibacteria and Arthrobacter have been used to produce amino acids and other products. Despite their great aconomic importance, perhaps due to uncertainties on their taxonomic position, work on the more fundamental aspects of their genetics has been limited. It is the current lack of basic knowledge that limits the application of recombinant DNA technology to Corynebacterium and related bacteria.

Procedures for cloning genes into E. coli are well known and the recombinant vector produced therein are well

characterized. The E. coli systems have the disadvantage that genese from some Gram-positive organisms, e.g., Bacillus, do not express well in the well characterized Gram-negative E. coli host. Thus, it would be advantageous to provide a suitable Gram-positive host-vector system, particularly when cloning genes from Gram-positive organisms. In addition, it would be desirable to provide a transformation procedure wherein the shuttle DNA vector can be introduced efficiently into bacterium cells capable of expressing the DNA vector.

In order to develop coryneform bacteria into more useful industrial organisms for applications and for a more broad based understanding of their basic genetics, their vector DNA molecules containing the essential genetic elements for their maintenance in Corynebacterium and carrying easily identifiable markers have been constructed, i.e., drug resistance to follow its presence in the cell.

In addition, an improved transformation system has been developed by which isolated or constructed genes can be reintroduced in the intact bacterium capable of expressing its genes, i.e., coryneform bacteria. It has been found that very high transformation efficiencies can be obtained when host cells are grown in glycine, in the absence of a chelating agent and are treated such as by agitation to avoid cell clumping.

The chimeric plasmids of the invention represent valuable intermediates for the development of improved host-vector systems. The process for introducing genetic information of interest into plasmids is well known in the art and such processes can be used to insert genes coding for various proteins, e.g., somatostatin, rat proinsulin, interferon, proteases, amino acid biosynthetic enzymes, antibiotic biosynthetic enzymes and enzymes for surface active

materials, immune stimulators and genes for excretion of biological products.

This invention recognizes the value and utility of genetic systems available with gram-negative bacteria such as Escherichia coli. Chimeric plasmids that serve as shuttle vectors between Escherichia coli and coryneform bacteria have been constructed. They can be used for a variety of biological processes including manufacture of many types of proteins. Other chemicals such as amino acids, surface active agents and polysaccharides could also be manufactured using the technology made available by this invention.

The invention can be utilized in several different ways. For example, microorganisms containing the chimeric Corynebacterium plasmids can be obtained and the plasmids can be isolated from the cells, e.g., the cells can be lysed and the components of the cell wall can be separated from residual cellular components soluble in the lysate. The chimeric plasmid can then be isolated from the lysate. The host cells can be lysed by contacting the cells with a suitably hypotonic medium and/or the cells are grown in the presence of glycine followed by exposing the cells to lysozyme and SDS. Separation of cell wall components can be accomplished simply by centrifugation at from 10.000 to 15.000 rpm, and the plasmid can be isolated from the lysate in a density gradient centrifugation. The plasmid fraction can be separated from the chromosomal DNA and other components by use of a syringe.

It has been found that unexpectedly high transformation efficiency is attained when the cells are grown in lysine, the cells are treated in the absence of a chelating agent such as EDTA and the cells are agitated during the transformation process. For example, the digested plasmid can be treated by well known techniques to make the ends

cohesive with those of the DNA fragment containing the gene to be inserted. The plasmid residue is mixed with the DNA fragment carrying the gene of interest, and the plasmid and gene sequences are allowed to anneal, followed by ligation. Subsequently, the plasmid modified by insertion of the new polynucleotide sequence can be used to transform either a Corynebacterium species and/or bacteria from a second genus which contributed a plasmid fragment forming part of the chimeric plasmid. Transformation is accomplished by treating the host cells to render them competent including growing the cells in glycine and in the absence of a chelating agent, followed by mixing the plasmid with the host cells and incubating the mixture to allow the plasmid to transform the host cells. The glycine in the growth medium is utilized at a level that improves cell growth rate but not at such a high concentration at which growth rate is halted. Generally suitable concentrations are between about 0,5 and 5%, preferably between about 1 to 2% w/v based upon the volume of the growth medium.The glycine can be used either alone or in admixture with other cell wall biosynthesis inhibiting compositions such as antibiotics such as penicillin or fatty acid biosynthesis inhibitors, such as cerulenium or the like.

The glycine composition and cells are contacted after the initial growth of cells in an initial growth medium. The glycine reduces all growth by weakening the cell wall. Agents that do not function as does glycine including gramicidine, nystatin, cetylpyridinium chloride and amino acids such as tryptophan or tyrosine. It has been found that reduced transformation efficiencies are obtained when the cells are grown in amino acids which do not include glycine. Where the host cell is a Corynebacterium organism or a coryneform cell is made competent by forming a protoplast as described above. Protoplasts are then transformed by mixing them with the chimeric plasmid carrying the inserted

polynucleotide sequence while maintaining an osmotically stable environment. The mixture is incubated for a sufficient time to allow for transformation of the protoplast, e.g., from 5 to 90 mins. at 37°C. The transformed protoplast is then incubated to allow regeneration of the cell wall. Incubation generally requires from 24 to 48 hours at 30 to 37°C. and is carried out in an osmotically stable environment as described above.

The invention can also be utilized by constructing the chimeric plasmid from plasmids obtained from coryneform bacteria and from at least one other species of microorganism, e.g., a Bacillus species. Coryneform bacteria refers to all bacteria classified in the following genera:

| | |
|---|---|
| Corynebacterium | Microbacterium |
| Arthrobacter | Brevibacterium |
| Cellulomonas | Mycobacterium |
| Curtobacterium | Nocardia |

"Coryneform Bacterium", edited Bousfield et al, published Academic Press, London. This invention provides unique and novel DNA sequences that contain information for chimeric plasmids that will replicate in a plurality of coryneform bacteria. These composition has the advantages that allows one to perform genetic engineering and strain combustion procedures in coryneform bacteria in an efficient manner. The chimeric plasmid is constructed by digesting the plasmid from coryneform bacteria with a restriction endonuclease; digesting the plasmid obtained from the non-Corynebacterium organism with a restriction endonuclease; if necessary, treating one or both of the plasmid digests to make the ends of the fragments complementary, mixing the restriction endonuclease digestion products of the two plasmids and incubating in the presence of a ligase. The constructed chimeric plasmid can be isolated as described above and further digested with a restriction endonuclease to allow

insertion of a foreign polynucleotide sequence coding for a desired protein, e.g., an antibiotic biosynthetic enzyme, amino acid biosynthetic enzyme, somatostatin, etc. The resulting chimeric plasmid carrying the inserted genetic information can then be used to transform a Corynebacterium protoplast or cells of a competent non-Corynebacterium organism from the genera (and preferably species) supplying one of the ligated plasmids.

Suitable non-coryneform bacteria organisms for the use in the invention include, but are not limited to, the following:

| | |
|---|---|
| Bacillus | Bifidobacterium |
| Staphylococcus | Peptostreptococcus |
| Streptococcus | Clostridium |
| Lactobacillus | Escherichia |
| Streptomyces | Pseudomonas |

The following microorganisms utilized in the invention have been deposited with and are available from the permanent collection of the Northern Regional Research Laboratory, U.S. Department of Agriculture, Peoria, Illinois, U.S.A.

A. Corynebacterium glutamicum, NRRL No-B-15304, a species containing the pSR1 plasmid described in Example 1.

B. Bacillus subtilis, NRRL No. B-15306, a species containing the pBD10 plasmid described in Example 1.

C. Bacillus subtilis, NRRL No. B-15307, a transformed species containing the pHY416 hybrid plasmid described in Example 2.

D. Corynebacterium glutamicum, NRRL No. B-15302, a transformed species containing the pHY416 hybrid plasmid described in Example 2.

E. Corynebacterium glutamicum, NRRL No. B-15301,

the species for transformation in Example 3,4, and 5.

F. Corynebacterium glutamicum, NRRL No. B-15743, a transformed species containing the pW111 hybrid plasmid described in Example 1.

G. Corynebacterium glutamicum, NRRL No. B-15756, a transformed species containing the pW112 hybrid plasmid described in Example 1.

H. Corynebacterium glutamicum, NRRL No. B-15744, a transformed species containing the pWS211 hybrid plasmid described in Example 2.

I. Corynebacterium glutamicum, NRRL No. B-15745, a transformed species containing the pW212 hybrid plasmid described in Example 2.

J. Escherichia coli, NRRL No. B-15746, a transformed species containing the pWS51 hybrid plasmid described in Example 1.

K. Escherichia coli, NRRL No. B-15747, a transformed species containing the pWS52 hybrid plasmid described in Example 1.

L. Escherichia coli, NRRL No. B-15748, a transformed species containing the pWS53 hybrid plasmid described in Example 1.

M. Escherichia coli, NRRL No. B-15749, a transformed species containing the pWS111 hybrid plasmid described in Example 1.

N. Escherichia coli, NRRL No. B-15750, a transformed species containing the pWS112 hybrid plasmid described in Example 1.

O. Escherichia coli, NRRL No. B-15751, a transformed species containing the pWS531 hybrid plasmid described in Example 1.

P. Escherichia coli, NRRL No. B-15752, a transformed species containing the pWS201 hybrid plasmid described in Example 2.

Q. Escherichia coli, NRRL No. B-15753,

a transformed species containing the pWS202 hybrid plasmid described in Example 2.

R. Escherichia coli, NRRL No. B-15754, a transformed species containing the pWS211 hybrid plasmid described in Example 2.

S. Escherichia coli, NRRL No. B-15755, a transformed species containing the pWS212 hybrid plasma described in Example 2.

Additionally, the invention utilizes the following organisms already deposited with the American Type Culture Collection of Rockville, Maryland, U.S.A.

A. Corynebacterium glutamicum, ATCC No. 13059, the species used for transformation in Example 4.

B. Brevibacterium lactofermentum, ATCC No. 13869, B. ammoniagenes, ATCC 13746, B.glutamigenes, ATCC 13747, B. roseum, ATCC 13825, B. flavum, ATCC 13826, B. ketoglutamicum, ATCC 21533, B. flavum, ATCC 13826, B. flavum, ATCC 15940 used in Example 1

C. Escherichia coli, HB101, the species used for transformation in Example 2.

All of these organisms have been deposited with the instruction that they be made available to the general public as of the date of issuance of the first patent based upon this application.

Description of Drawings:

Figure 1 Restriction Map of pWS101,

Figure 2 Constructions between pBR325 and pWS101,

Figure 3 Potential E. coli /Coryneform Shuttle Vectors Containing pBR325, pWS101 and pBD10,

Figure 4 Potential E. coli /Coryneform Shuttle Vectors Constructed between pBR322 and pHY416,

Figure 5 Transformation Protocol.

Description of Tables:

Table 1 Plasmid Screening of Brevibacterium strains,

Table 2 Restriction Sites in pWS101,

Table 3 Restriction Data from Double Digestions of pWS101,

Table 4 Transformation Frequencies of E. coli / Coryneform Shuttle Vectors in NRRL No. B-15301,

Table 5 Transformation Efficiency Represents Number of Kanamycin Resistant Colonies Obtained per μg DNA.

## Example I
## Development of Shuttle Vectors

I. Characterization of the Plasmid pWS101 from Brevibacterium lactofermentum

i. Isolation

A small plasmid from the Brevibacterium lactofermentum ATCC No. 13869 was identified using a conventional plasmid screening and isolation procedure. Other species of Brevibacterium were also examined and the results are summarized in Table 1.

The protocol set forth below was successfully used to isolate pure pWS101 DNA from Brevibacterium lactofermentum ATCC No. 13869. Several features of this protocol deserve special notice. First, the lysozyme treatment is done in the presence of PEG-8000 instead of sucrose because PEG increases the effectiveness of lysozyme. Secondly, lysozyme-treated cells are spun down and resuspended in a medium without an osmotic stabilizer such as sucrose or PEG prior to SDS lysis. As with C. glutamicum, this step appears to be essential for complete cell lysis.

Thirdly, a phenol extraction was performed on the cleared

lysate supernatant prior to PEG precipitation and CsCl density centrifugation. This step was inserted to remove most of the protein which had not been precipitated by the addition of 5 M NaCl and results in a PEG precipitate which is readily resuspended and CsCl gradients which give clear separation of chromosomal and plasmid DNA

Table 1. Plasmid Screening of Brevibacterium Strains

| Strain Number | Species | Plasmid[1] |
|---|---|---|
| ATCC 13746 (AS604) | B.ammoniagenes | -- |
| ATCC 13747 (AS605) | B.glutamigenes | -- |
| ATCC 13825 (AS606) | B.roseum | -- |
| ATCC 13826 (AS607) | B.flavum | -- |
| ATCC 13869 (AS608) | B.lactofermentum | + (4.4 Kb) |
| ATCC 21533 (AS618) | B.ketoglutamicum | -- |
| ATCC 15940 (AS619) | B.flavum | -- |

[1] A "+" indicates the presence of a plasmid in the strain. A "-" indicates that no plasmid DNA was detected in the strain.

Isolation of pWS101 DNA from Brevibacterium lactofermentum ATCC No. 13869

1 liter of ATCC No. 13869 cells grown to early stationary phase in Luria broth or Brain Heart Infusion broth

Wash once in 1/10 volume of 10 mM Tris (pH 8,0)

Resuspend in 30 ml 30 mM Tris (pH 8,0)

Add 60 ml 24% PEG-8000 (w/v)

Add 10 ml 50mg/ml lysozyme in 30 mM Tris (pH 8,0) Incubate cells at 37°C. for 1 hour with occasional mixing

Centrifuge cells and resuspend in 30 ml 100 mM Tris/10 mM EDTA (pH 8,0)

Divide cells equally into two SS34 tubes and add 1,5 ml 0,5 M EDTA per tube

Add 2 ml 10% SDS per tube and invert gently several times

Incubate tubes at 37°C. for 15 minutes or until lysis with occasional inversion

Add 5 ml 5 M NaCl per tube and invert several times

Place tubes on ice 2-6 hours

Spin at 15.000 rpm for 30 minutes

Recover supernatant and extract once with an equal volume of phenol (pH 7,0 and equilibratet against 100 mM Tris)

Extract aqueous phase twice with chloroform

Add ,31 volumes of 42% PEG-8000 to the aqueous phases and place at 4µC. overnight

Resuspend PEG precipitate in 50 mM NaCl/30mM Tris/5 mM EDTA (pH 8,0) and subject to CsCl-ethidium bromide density centrifugation

Collect lower plasmid band

Remove ethidium bromide by 5 extractions with isopropanol (saturated against 5 M NaCl)

Dialyze against 10 mM Tris/1 mM EDTA (pH 8,0)

Ethanol precipitate

Although this procedure gave pure plasmid DNA, the yields were quite small (25-50 µg DNA/liter of cells).

B. Restriction Mapping of pWS101

Pure pWS101 DNA was digested with a variety of restriction endonucleases, most of which have a six base recognition sequence. pWS101 contains no restriction sites for the following enzymes: Eco RI, Pvu II, Sal I, Pst I, Bam HI, Xho I, Bgl II, Kpn I, Xma I, and Sst I.Table 2 shows the data for the six enzymes which have been shown to restrict pWS101 thus far. Four enzymes, Bcl I, Hpa I, Hind III, and Xba I all cut pWS101 once. Bcl I, Hind III, and Xba I produce "sticky" ends while Hpa I produces "blunt" ends. BstE II and Acc I produce two and three restriction fragments, respectively.

The positions of these restriction sites have been mapped relative to one another by measuring the sizes of the fragments produced by all possible combinations of two enzymes. This data is presented in Table 3 and the resulting restriction map is shown in Figure 1. In general, the calculated sizes of pWS101 fall in the range of 4,2-4,6 Kb. The major exceptions to this rule are digestions involving Acc I which yield calculated sizes less than 4,15 Kb. This could either be due to the inability to detect an additional small Acc I fragment or to a systematic error in the

measurement of the sizes of restriction fragments under 2,0 Kb.

The best estimate of the size of pWS101 is 4,4 Kb and is based both on the calculated sizes from Tables 1 and 2 and on the observation that the mobility of linear pWS101 is just less than that of the 4,371 Kb standard fragment from Hind III-digested DNA. The restriction map in Figure 1 has been constructed using this 4,4 Kb estimate. The relative positions of the Hind III and Acc I 9C) sites and Bcl I and Acc I (b) sites are not yet known with a high degree of confidence.

Use of pWS101

The original concept for screening Brevibacterium sp. strains for plasmids was predicated on the hypothesis that such plasmids could also replicate in other coryneform bacteria, expecially C. glutamicum. Thus, these plasmids would provide an additional source of replicons for the construction of a diverse array of C. glutamicum vectors which may not only differ in the kinds of useful restriction sites available but also in their plasmid compatibilites. The construction of the pWS101 restriction map (Figure 1) made it possible to test whether this particular plasmid will be useful in the development of recombinant DNA technology in C.glutamicum.

pWS101 is a potentially valuable vector component because it has at least four different single restriction sites, three of which yield "sticky" ends and one which produces "blunt" ends. Another fortuitous aspect of pWS101 is that these four restriction sites are well distributed over the plasmid so that at least some of them are available for cloning purposes without affecting its replication function(s). Using several approaches with pWS101, the construction of

coryneform vectors is now described.

A two step strategy was employed which offered the advantage of being able to identify the desired clones by phenotypic screening rather than direct plasmid screening. As described below this approach has proven quite successful.

The first cloning step was the insertion of pWS101 into pBR325, an E. coli vector conferring $Ap^r$, $Cm^r$ and $Tc^r$. Although pBR325 (5,4Kb) is larger than pBR 322 (4,4 Kb), it was chosen as the E.coli vector because it has three drug resistance genes, all of which contain at least one unique restriction site for insertional inactivation.

pWS101 was inserted into pBR325 in two different ways:
a) pBR 325 and pWS101 were linearized by HindIII digestion and then ligated together, and
b) pBR325 was linearized by BamHI digestion, pWS101 was linearized by BclI digestion, and the two plasmids were ligated together. In the latter case, two different enzymes could be used because they produce the exact same "sticky" ends. Following the ligation reaction, each mixture was transformed into E.coli HB101 and $Ap^r$ transformants were selected. Transformants from both ligation mixtures were screened for a $Tc^s$ phenotype since insertion of foreign DNA at the BamHI or HindIII sites of pBR325 inactivate the Tc gene. Approximately 10-20% of all $Ap^r$ transformants from both cloning experiments were $Tc^s$. Both orientations of pWS101 inserted into pBR325 via their common HindIII sites werde obtained. These plasmids have been designated pWS51 and pWS52 and their structures are shown in Figure 2. This plasmid has been named pWS53 and its structure is also shown in Figure 2.

Having inserted pWS202 into pBR325 it was then necessary to add a selectable marker to these vectors which is capable of

expression in C. glutamicum. The Km gene from pBD10 represented the best candidate since it is known to be expressed in C.glutamicum NRRL No. B-15340 (U.S.Patent application No. 489,298, filed April 28,1983). Thus far, the Km gene from pBD10 has been successfully inserted into both pWS51 and pWS53 to give the potential E.coli |coryneform shuttle vectors shown in Figure 3. pWS111 and 1pWS112 (Figure 3) were produced by linearizing pBD10 DNA with BamHI and ligating it into the single BamHI site of pWS51. Since pWS51 already contained an inactivated Tc gene it was impossible to detect the insertion of pBD10 into pWS51 by insertional inactivation of the Tc gene. However, we were able to distinguish the desired clones from the reclosed vector based on their increased $Km^r$ and $Cm^r$. Approximately 10% of all $Ap^r$ transformants from this cloning experiment were also $Km^r$ and $Cm^r$. Restriction analysis of plasmids from $Ap^r$ $Km^r$ $Cm^r$ transformants revealed the two expected classes of plasmids represented by pWS111 and pWS112 in Figure 3.

Although pWS111 and pWS112 presumably contained all the components necessary for a useful E.coli /coryneform shuttle vector, it was not certain that the coryneform replication functions of pWS101 were still intact. As a result an analogous vector starting with pWS53 in which pSW101 sequences were interrupted at the BclI site which is 1,85 Kb from the HindIII site was constructed.

This latter construction was somewhat more complicated than the previously described experiments because pWS53 and pBD10 are not known to share any unique restrictions sites. The strategy adopted involved EcorRI digestion of pWS53 to yield full length linear molecules with "sticky" ends. The four basis of single stranded DNA at each 5' and were filled in by the action of E. coli DNA polymerase using the procedure described by Maniatis et al. (1982) "Molecular Cloning, A Laboratory Manual," Cold Spring Harbor Laboratory.The

formation of blunt ends was quite efficient since the majority of the $Ap^r$ transformants obtained after reclosure of these DNA molecules by T4 DNA ligase were $Cm^s$. (Note that the EcoRI site is within the Cm gene of pBR325 and that transformants arising from ligation of blunt end molecules contain a 4 bp insert which inactivates the Cm gene.)

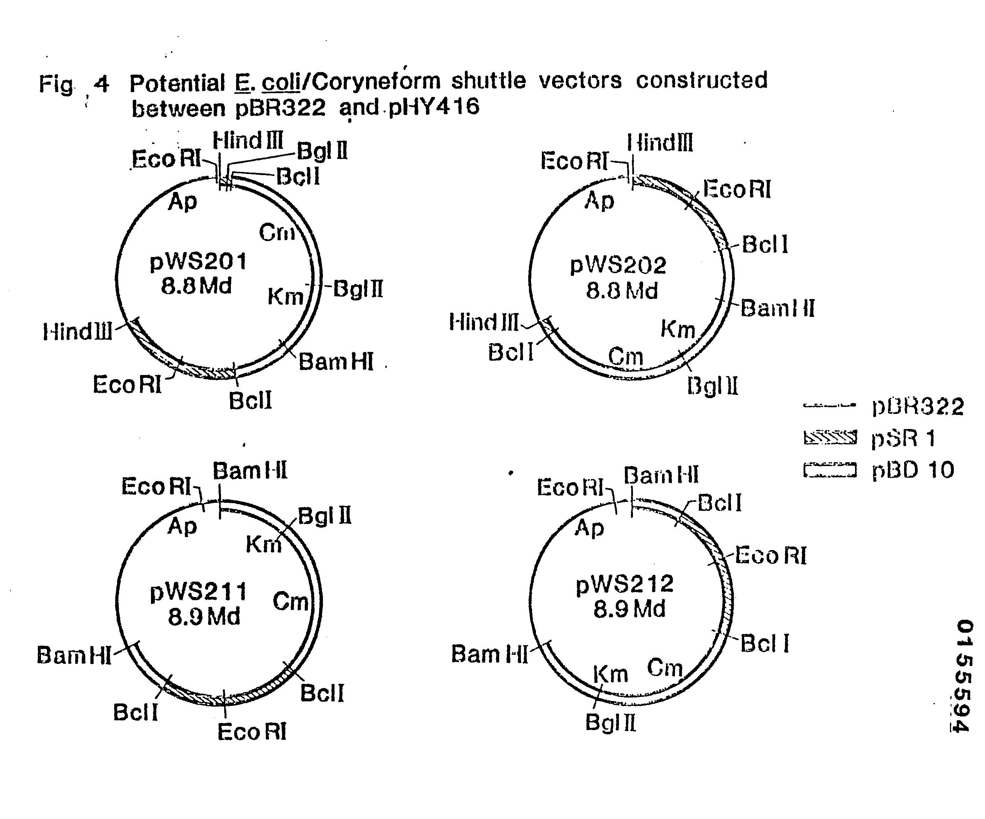

Next, pBD10 was partially digested with HaeIII, a restriction endonuclease with a 4 bp recognition sequence which produces blunt ends. The partial digest was performed so that most of the pBD10 molecules were full length or nearly full length in size. The pBD10 DNA cut with HaeIII was then ligated to the blunt end molecules of pWS53 and transformed into E. coli HB101. $Ap^r$ transformants were selected and screened for the presence of the Km gene of pBD10. Plasmids from several $Ap^r$ $Km^r$ transformants werde analyzed but only one, pWS531 (Figure 3), was mapped in detail. Several characteristics of this plasmid are noteworthy. First, as predicted from their recognition sequences, the ligation of a HaeIII fragment to EcoRI-blunt ends generates two EcoRI sites as shown in Figure 3. Furthermore, since the EcoRI sites define the junctions of the two ligated fragments the smaller EcoRI fragment is equeal to the size of the inserted HaeIII fragment. The mobility of this fragment is indistinguishable from the mobility of linear pBD10 molecules so it can also be concluded that the entirety of pBD10 has been inserted into pWS53. All other mapping data is consistent with this conclusion. The HaeIII site at which pBD10 was cleaved during this construction is located between the Km and Cm genes of pBD10. Lastly, the phenotype conferred by pWS531 clearly shows that the Km and Cm genes of pBD10 are still functional. E. coli HB101 cells containing pWS5341 exhibit the same limites of $Km^r$ and $Cm^r$ as E. coli HB101 cells containing pWS201 (Figure 4) but are less $Cm^r$ than cells carrying the E. coli Cm gene present on pBR325.

Table 2 Restriction Sites in pWS101[a]

| Enzyme | Number of Sites | Fragment Size(s) | Calculated Size of pWS101 |
|---|---|---|---|
| AccI | 3 | 1,92 | 4,14 |
| | | 1,12 | |
| | | 1,1 | |
| Bcl | 1 | 4,25 | 4,25 |
| BstE II | 2 | 3,85 | 4,73 |
| | | 0,88 | |
| Hind III | 1 | 4,2 | 4,2 |
| Hpa I | 1 | 4,37 | 4,37 |
| Xba I | 1 | 4,35 | 4,35 |

[a] Standard restriction mapping techniques were employed. Approximately 0,5 μg plasmid DNA was digested with each enzyme in the appropriate buffer and the subjected to electrophoresis through 1,0% agarose gels. DNA fragments were visualized with ultraviolet light after staining with ethidium bromide. Sizes were calculated using Hind III digests of λ-DNA as standards.

Table 3 Restriction Data from Double Digestions of pWS101

| Enzymes | Fragment Sizes | Calculated Size of pWS101 |
|---|---|---|
| Hind III + Bcl I | 2,45 | 4,30 |
| | 1,85 | |
| Hind III + Xb I | 2,35 | 4,35 |
| | 2,0 | |
| Bcl I + Xba I | 4,0 | 4,43 |
| | 0,43 | |
| Hpa I + Hind II | 3,15 | 4,45 |
| | 1,13 | |
| Hpa I + Bcl I | 3,42 | 4,39 |
| | 0,97 | |
| Hpa I + Xba I | 3,85 | 4,46 |
| | 0,61 | |
| BstE II + Hind III | 3,15 | 4,61 |
| | 0,86 | |
| | 0,60 | |
| BstE II + Bcl I | 3,3 | 4,61 |
| | 0,88 | |
| | 0,43 | |
| BstE II + Xba I | 2,75 | 4,39 |
| | 0,86 | |
| | 0,78 | |
| BstE II + Hpa I | 2,0 | 4,21 |
| | 1,42 | |
| | 0,79 | |
| Acc I + Hind III | 1,92 | 4,14 |
| | 1,12 | |
| | 1,1 | |
| Acc I + Bcl I | 1,92 | 4,14 |
| | 1,12 | |
| | 1,1 | |

| | | |
|---|---|---|
| Acc I + Xba I | 1,92 | 4,13 |
| | 1,08 | |
| | 0,73 | |
| | 0,40 | |
| Acc I + Hpa I | 1,92 | 3,94* |
| | 1,08 | |
| | 0,94 | |
| | * | |
| Acc I + BstE II | 1,15 | 3,93 |
| | 1,1 | |
| | 0,79 | |
| | 0,57 | |
| | 0,42 | |

*There is a fourth fragment which would be predicted to be approximately 0,15-0,2 kg. Such a small fragment could not be detected on the agarose gels used. As a result, the calculated size of pWS101 is artificially low.

Example 2

Construction of E. coli/C. glutamicum Shuttle Vectors

i. Rationale

Since E. coli still has the most advanced recombinant DNA and genetic systems available, an E. coli shuttle system offers several advantages over the existing B. subtilis system. They are:

1) Routine manipulations such as DNA isolation are easier.
2) Plasmid transformation efficiency in E. coli is higher.
3) More versatile recombinant DNA vectors are available including expression vectors.
4) Better and more efficient methods of analyzing clones are available.
5) More and better characterized mutants exist in E. coli

which can be used to increase the stability of cloned segments of DNA or to aid in the selection of desired clones.

The major disadvantage of E. coli is that it is a gram-negative organism and C. glutamicum genes are less likely to be expressed in E. coli without providing a promoter. Nevertheless, there are examples of genes from gram positive organisms being expressed in gram-negative bacteria and the fact that the $Cm^r$ gene from B. subtilis does not function in C. glutamicum also indicates that C. glutamicum genes may not necessarily funtion in other gram (+) bacteria.

ii. Strategies

There are four requirements of any E. coli/C. glutamicum shuttle vector. These are:

1) the ability to replicate in E. coli,
2) the expression of a selectable marker (e.g., drug resistance) in E. coli,
3) the ability to replicate in C. glutamicum, and
4) the expression of a selectable marker in C. glutamicum.

pSR1, a cryptic plasmid isolated from C. glutamicum has already been cloned into the B. subtilis plasmid pBD10 to yield the B. subtilis/C. glutamicum shuttle vector pHY416 (Sinskey et al., U.S. Patent Application No. 489,298, filed April 28,1983). We have previously shown that pHY416 contains a functional C. glutamicum replicon and a selectable marker ($Km^r$) which is expressed in C. glutamicum. As a result, the simplest approach to constructing an E. coli(C. glutamicum shuttle vector was to clone pHY416 into a suitable E. coli vector. For this purpose we chose pBR322 because it had single restriction sites identical to some of

those in pHY416.

Two separate cloning strategies were utilized to insert pHY416 into pBR322. In the first method, PBR322 and pHY416 were both cleaved with Hind III, mixed and ligated together, and $Ap^r$ transformants were selected. The desired clones were easily obtained because insertion of foreign DNA at the HindIII site of pBR322 usually inactivates the Tc gene. In addition, the Km and Cm genes of pBD10 are expressed in E. coli at sufficient levels to identify clones containing pHY416 by screening transformants for resistance to Km or Cm. Approximately 1% of the $Ap^r$ transformants obtained had an Ap $T^s$ $Km^r$ $Cm^r$ phenotype.

The plasmid DNA from several of these clones was analyzed and generally fell into the two classes represented by pWS201 and PWS202 in Figure 4. pWS201 and pWS202 are simply different orientations of pHY416 inserted into pBR322. It should be noted that a small HindIII fragment (0,3 Kg) derived from pSR1 was deleted from pHY416 during the construction of pWS201 and pWS202.

An examination of the restriction maps of pWS201 and pWS202 (Figure 4) also shows that there is an EcoRI site not predicted by the restriction maps of pBR322, pBD10, and pSR1. Restriction analysis of pSR1, pBD10, pHY416 and pWS201 has led to the conclusion that an EcoRI restriction site was formed during the construction of pHY416 and that this new site is located within pSR1 sequences as shown in Figure 4.

The second method by which pBR322/pHY416 hybrid plasmids were produced was identical to the first method except the two plasmids were cleaved by BamHI endonuclease instead of HindIII prior to ligation. Selection and screening procedures were the same since insertion of foreign DNA at the BamHI site of pBR322 inactivates the Tc gene. The

obvious advantage of this procedure is that pHY416 is opened at the BamHI site derived from pBD10 sequences. As a result, it is almost certain that the C.glutamicum replicon is not lost during this construction.

Approximately 5% of the $Ap^r$ transformants from the BamHI cloning of pHY416 into pBr322 had a $Tc^s$ $Km^r$ $Cm^r$ phenotype. Plasmids from eight of these transformants were analyzed by restriction analysis. Three were very large plasmids containing more than one copy of pBD10 or pBR322. However, the other five plasmids had restriction maps expected for pBR322/pHY416 hybrid plasmids. Three plasmids had the structure shown for pWS211 in Figure 4 while the other two plasmids had pHY416 inserted into pBR322 in the opposite orientation. The latter plasmids are designated pWS212 in Figure 4.

## Example 3

Transformation of C. glutamicum with E. coli/coryneform shuttle vectors

Table 4 summarizes the results from an experiment in which C. glutamicum strain NRRL No. B-15301 was transformed with several of the E. coli /coryneform shuttle vectors described above. The major conclusions and hyptheses suggested by these results are the following:

1) pWS211, pWS212, pWS111, and pWS112 are all functional E. coli / coryneform shuttle vectors.
2) The replication functions of the Brevibacterium lactofermentum plasmid, pWS101, function in C. glutamicum since transformants of pWS111 and pWS112 were obtained.
3) Transformants of pWS201 and pWS531 were not detected. This result is propably due to inactivation of the

replication functions of pSR1 and pWS101 during the construction of pWS201 and pWS531, respectively. If so, then the HindIII sites of pSR1 and the BlcI site of pWS101 are located in regions essential for plasmid replication and maintenance.

4) The transformation frequencies of the E. coli / coryneform shuttle vectors are much lower than that of pHY416 isolated from C. glutamicum. This observation could result from restriction of foreign DNA by C. glutamicum or could arise from other differences such as plasmid sizes.

Table 4

Transformation Frequencies of E. coli / coryneform shuttle vectors in NRRL No. B-15301[a]

| Plasmids | Transformant/μg DNA |
|---|---|
| pHY416 | 1.800 |
| pWS201 | 0 |
| pWS212 | 8 |
| pWS111 | 2 |
| pWS112 | 2 |
| pWS531 | 0 |

[a] Transformation experiments were performed using the protocol described herein. For pHY416, 3 μg plasmid DNA was used whereas 30-50 μg plasmid DNA was added to transformation mixtures containing E. coli / coryneform shuttle vectors.

Example 4 - Transformation Procedure

a. Bacterial Strains and Culture Media Transformation Procedure

C.glutamicum NRRL No. B-15301 is used as the recipient cell in most of the transformation experiments carried out. Other strains of Corynebacterium can be used. For example, C. glutamicum strain ATCC 13059 can be used. Corynebacterium glutamicum NRRL No. B-15301 is a natural rifampicin resistant variant of ATCC 13059 that was isolated by spreading fresh cultures of ATCC 13059 on rifampicin plates containing 1 µg/ml of the antibiotic. Corynebacterium glutamicum NRRL No. B-15301 is routinely grown in LB broth containing 5-10 µg/ml rifampicin at 30°C. with shaking.

b. Plasmid Preparation and Purification

pHY416 is the chimeric plasmid containing a C. glutamicum origin of replication from plasmid (pSRI) with drug resistant markers from Bacillus subtilis (pBD10). pHY416 was isolated from C. glutamicum by lysozyme-SDS lysis followed by CsCl-EtBr density gradients of the cleared lysate. The lower density band containing supercoiled plasmid is isolated from the centrifuge tubes by puncturing the side and purified by washing with isopropanol saturated with buffer and precipitating the plasmid DNA with 70% EtOH and finally dialyzing for 24-36 hours against Tris EDTA buffer.

The final plasmid preparation is stored in Tris-EDTA (10 mM; 1 mM) buffer, pH 7,5 at 1 to 1,5 µg/DNA µl concentration. Reagents used in experiment are prepared as follows:

SMMC

Sorbitol - 0,5 M
Maleate - 0,02 M
$MgCl_2$ - 0,02 M
$CaCl_2$ - 0,02 M pH 20

SB plates - Nutrient plates containing sorbitol as an osmotic stabilizer

| | |
|---|---|
| Yeast extract | - 5,0 gm/l |
| Tryptone | - 10,0 gm/l |
| NaCl | - 10,0 gm/l |
| Sorbitol | - 0,5 M |
| $MgCl_2$ | - 0,02 M |
| $CaCl_2$ | - 0,02 M |
| Agar | - 10,0 gm/l |

Equeal volumes of 1 M sorbitol (pH 7,0) and 2X nutrient both-agar were sterilized separately and mixed before pouring the plates. Note that $CaCl_2$ should be added after agar is cooled down.

SBK plates - SB plates containing kanamycin at 15 μg/ml concentration.

SB Solution

| | | |
|---|---|---|
| Yeast extract | - 5,0 gm/l | |
| Tryptone | - 10,0 gm/l | |
| NaCl | - 10,0 gm/l | |
| Sorbitol | - 0,5 M | pH 7,0 |
| $MgCl_2$ | - 0,02 M | |
| $CaCl_2$ | - 0,02 M | |

PEG Solution - 50% polyethyleneglycol (M.W. 12.000 M.W. 3.350 or 8.000) dissolved in 0,5 M sorbitol solution.

The protocol for the preparation of cells for transformation and conditions of transformation is set forth below. Corynebacterium glutamicum NRRL No. B- 15301 grown in the

presence of glycine followed lysozyme treatment result in consistently higher frequencies of transformation, whereas growth in amino acid composition not containing glycine resulted in low transformation efficiencies.

Figure 6

Transformation Protocol

One loop of NRRL No. B-15301 on LBR plate into 10 ml LB broth containing 10 µg/ml rifampicin and 0,2% glucose.

Incubate the tube on shaker at 30°C. overnight

Transfer 100 µl of overnight culture into 10 ml LB broth containing 10 µg/ml rifampicin, 0,2% glucose and 2 % glycine

Incubate it on shaker at 30°C. for 15 hours

Harvest cells by centrifugation

Wash cells once with SMMC buffer and by centrifugation

Resuspend pellet in 1 ml of SMMC containing 2,5 mg/ml lysozyme

Incubate the cell suspension at 30°C. for 100 min with agitation

Harvest cells by centrifugation

Resuspend cells in 10 ml of SMMC

Take 0,3 ml aliquote to 16 x 100 mm tubes

Add 5 μl of 1 M glucose in 0,5 M sorbitol (pH 7,0)

Add approx. 3 μg pHY416 DNA prepared in 0,5 M sorbitol

Hold the tube at 42°C. for 3 min

Add 0,7 ml of 30 - 50% PEG solution (M.W. 3.350 or 8.000) prepared in 0,5 M sorbitol and mix gently to obtain uniform suspension

Add 2 ml SB broth

Incubate the tube at 30°C. for 3 hours

Plate 100 μl on SBK plates

Incubate the plate at 30°C. for 3 - 10 days.

Example 5

Cloning of Recombinant DNA in C. glutamicum

a. Rationale

The procedure was developed to show whether recombinant molecules of Bam H1 cut chromosomal DNA from C. glutamicum ligated into the Bam H1 site of pHY82 could be transformed into C. glutamicum and under what conditions transformation could be demonstrated.

b. Materials and Methods

Chromosomal DNA was isolated from Corynebacterium glutamicum strain NRRL No. B-15301 and purified on a CsCl gradient. Plasmid pHY82 was isolated by a cleared lysate and purified

on CsCl gradients. Twenty µg of pHY82 was cut with Bam H1 as was chromosomal DNA. Bam H1 restriction was carried out in buffers described in Davis et al. (1980), "Advanced Bacterial Genetics, A Manual for Genetic Engineering," Cold Spring Harbor Laboratory, New York. Elektrophoresis through a 0,7% agarose gel demonstrated complete cutting of the plasmid and the generation of Bam cut chromosomal DNA fragments with an average size of 4kb. Six µg of the Beam cut pHY82 was treated with calf intestine phophatase (CIP) in buffers described in Maniatis et al. (1982), "Molecular Cloning, A Laboratory Manual," Cold Spring Harbor Laboratory, New York. Both CIP and nonCIP treated DNA was heated at 68°C. for 20 min. followed by a phenol extraction, chloroform extraction and ethanol precipitation in order to inactivate and remove the enzymes used. Ligations were carried out in buffers described in Maiatis et al. (1982), "Molecular Cloning, A Laboratory Manual," Cold Spring Harbor Laboratory, New York, with plasmid concentrations of 0,2 µg/µl and an approximately equeal molar amount of chromosomal DNA when appropriate. One third of each DNA was analyzed on an 0,7% agarose gel to check CIP treatment and ligation while the reminder of each DNA sample (2 µg) was used to transform a histidine auxotroph of C. glutamicum strain NRRL No. B-15301. Transformation was accomplished as described above using PEG (3.500) induced transformation of lysozyme treated cells. Cells were plated onto SB plates containing 15 µg/ml kanamycin in order to select transformants.

Fifteen random transformants from the CIP treated plasmid religated in the presence of Bam cut DNA from NRRL No. B-15301 were subject to analysis. Plasmid DNA was isolated from cells grown in 10 mls of LB containing 15 µg/ml of kanamycin. These plasmids were subjected to Bam H1 restriction and electrophoresed through an 0,7% agarose gel.

Result

Agarose gel analysis demonstrated that both ligation and CIP treatment of DNA was sucessful. Greater than 90% of the Bam cut DNA was seen to religate whereas there was no detectable ligation of pHY82 which had been both Bam restricted and terminal phosphates removed by CIP treatment. Approximately 50% of the Bam cut CIP treated plasmid was seen to ligate when Bam H1 generated fragments of C. glutamicum chromosomal DNA was added.

Transformation efficiencies of the variously treated DNAs is shown in Table 5.

Table 5. Transformation efficiency represents of kanamycin resistant colonies obtained per μg DNA.

| DNA | transformation efficiency |
|---|---|
| pHY82 | 15.000 |
| pHY82 + BamH1 | 15 |
| pHY82 + BamH1 + ligase | 1.425 |
| pHY82 + BamH1 + NRRL No. B-15301 DNA + ligase | 120 |
| pHY82 + BamH1 + CIP + ligase | 60 |
| pHY82 + BamH1 + CIP + AS019 DNA + ligase | 375 |

Fifteen random transformants from the CIP treated plasmid ligated in the presence of Corynebacterium NRRL No. B-15301 chromosomal DNA were checked for the presence of chromosomal DNA inserted into the Bam H1 site of pHY82. Twelve of the plasmids were able to be analyzed (the remaining three were uncut) demonstrated three which carried chromosomal DNA

inserted in the Bam H1 site of pHY82.

Transformants were checked for complementation of the histidine auxotrophy by replica plating onto minimal media plates containing 15 µg/ml of kanamycin.

The results show that recombinant DNA can be isolated in C. glutamicum using our PEG induced transformation and pHY82 as a vector.

March 6,1985
Eu 85 397 Gr/fr

Claims:

1. A process for transforming a chimeric plasmid into a bacterial host cells comprising:
   (a) treating the host cells with glycine and in the absence of a chelating agent to enable them to receive said chimeric plasmid; and
   (b) mixing the chimeric plasmid with the host cells and incubating the mixture to allow the plasmid to transform the host cells, said chimeric plasmid comprising the ligation product of a plasmid restriction fragment from at least two different species of microorganism.

2. A process as in Claim 1, wherein the host cells are gram-posititve.

3. A process as in claim 1 or 2, wherein the host cells are from the same genus as one of the organisms donating a restriction fragment to the chimeric plasmid.

4. A process a in Claim 1 to 3, wherein the host cells are a member of the genus Corynebacterium or of the genus Bacillus.

5. A process a in Claim 4, wherein the host cells are a species of Bacillus subtilis or of Corynebacterium glutamicum.

6. A process as in Claim 5, wherein the host cells are Bacillus subtilis NRRL B-15341 or Corynebacterium

glutamicum ATCC 13059 or Corynebacterium NRRL B-15301 or Brevibacterium flavum ATCC 15940.

7. A process a in Claim 1 to 3, wherein the bacterial host is a Corynebacterium protoplast and including the step of regeneration of the transformed protoplast.

8. A process as in Claim 7, wherein the host is a Corynebacterium glutamicum protoplast or a protoplast prepared from Corynebacterium ATCC 13059 or a protoplast prepared from Corynebacterium NRRL B-15301.

9. A process as in Claim 1, wherein the host is a Corynebacterium protoplast and the step of treating the host cells further comprises culturing the host cells at various times during growth in the presence of glycine and contacting the cells sequentially with lysozyme while in an osmotically stable environment.

10. A process for preparing Corynebacterium protoplasts comprising culturing said bacteria in the presence of glycine and contacting the cells with lysozyme while in an osmotically stable environment.

11. A process as in Claim 10, wherein the bacteria is Corynebacterium glutamicum especially Corynebacterium glutamicum ATCC 13059 or Corynebacterium glutamicum NRRL B-15301.

12. A chimeric plasmid capable of expression in at least one species of Corynebacterium and at least one species from a second genus of microorganisms comprising ligated restriction fragments from both of said species of bacteria.

13. A chimeric plasmid as in Claim 12, wherein the non-Corynebacterium species is a gram-positive organism.

14. A chimeric plasmid as in Claim 12 or 13, containing relevant fragments of a Corynebacterium glutamicum plasmid or of a Brevibacterium Plasmid or plasmid DNA from an organism of the genus Bacillus or plasmid DNA from an organism of the genus Escherichia.

15. A chimeric plasmid as in Claim 12 or 14, wherein the non-Corynebacterium plasmid is from Bacillus subtilis or from Escherichia coli.

Figure 1

Restriction Map of pWS 101

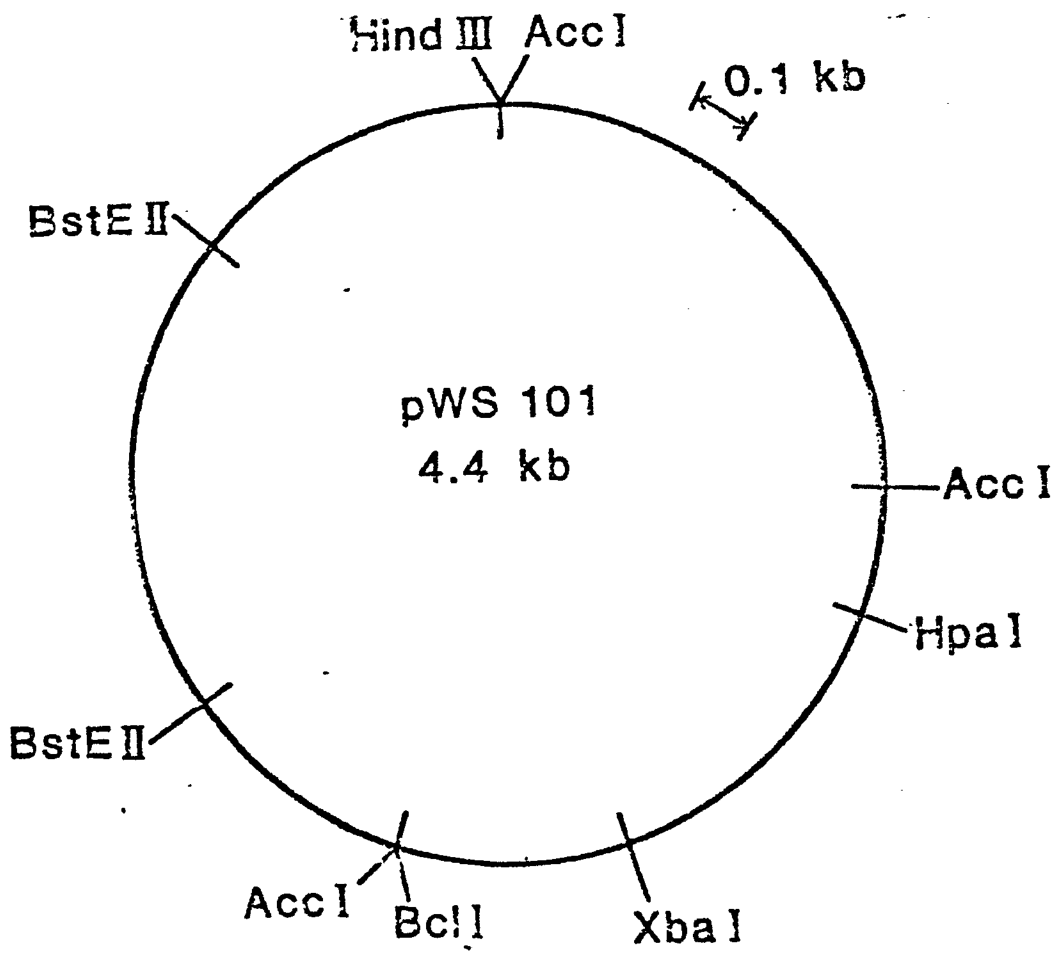

Fig 2 Constructions between pBR325 and pWS101

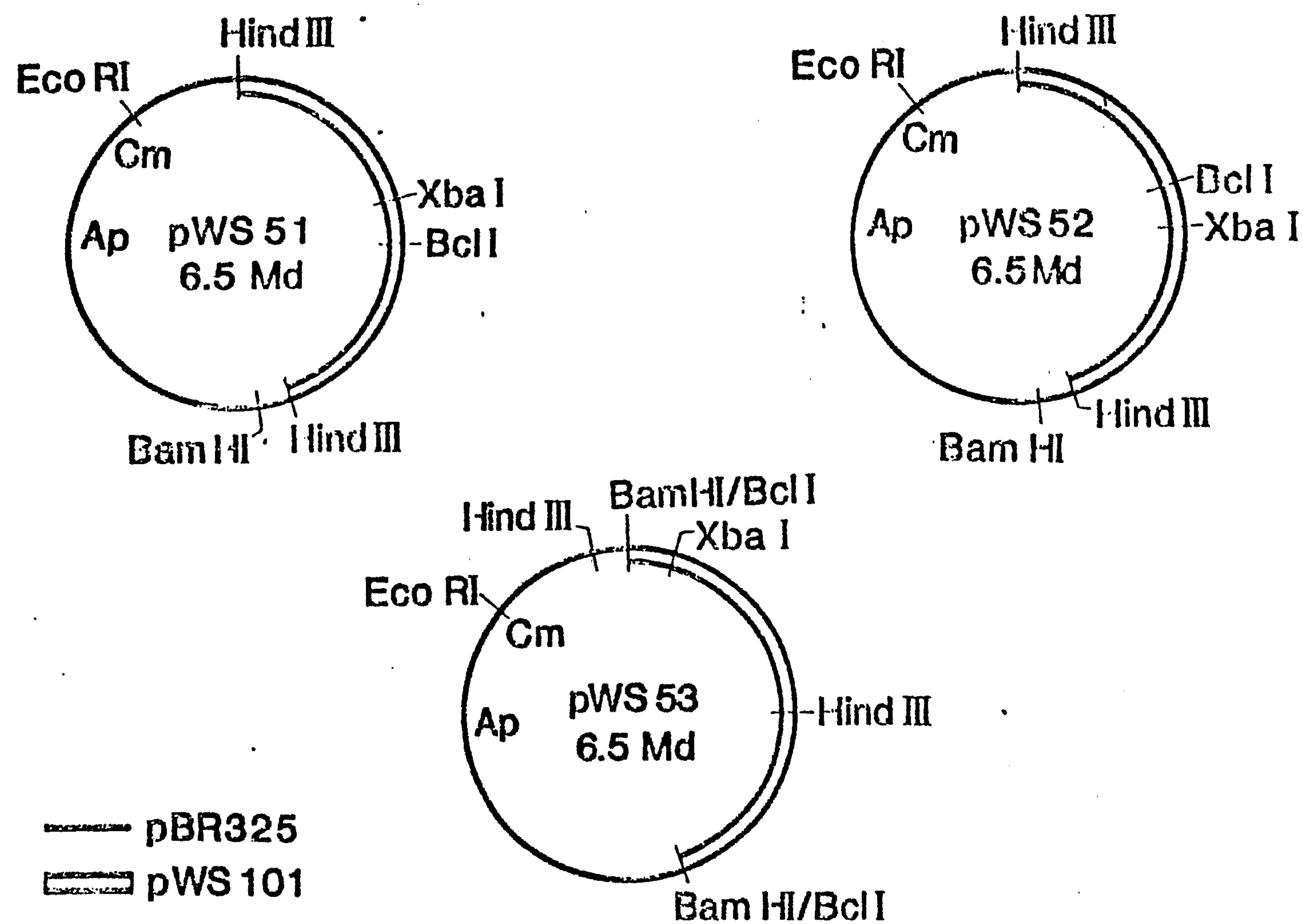

Fig. 3 Potential E. coli/Coryneform shuttle vectors containing pBR325, pWS101 and pBD10

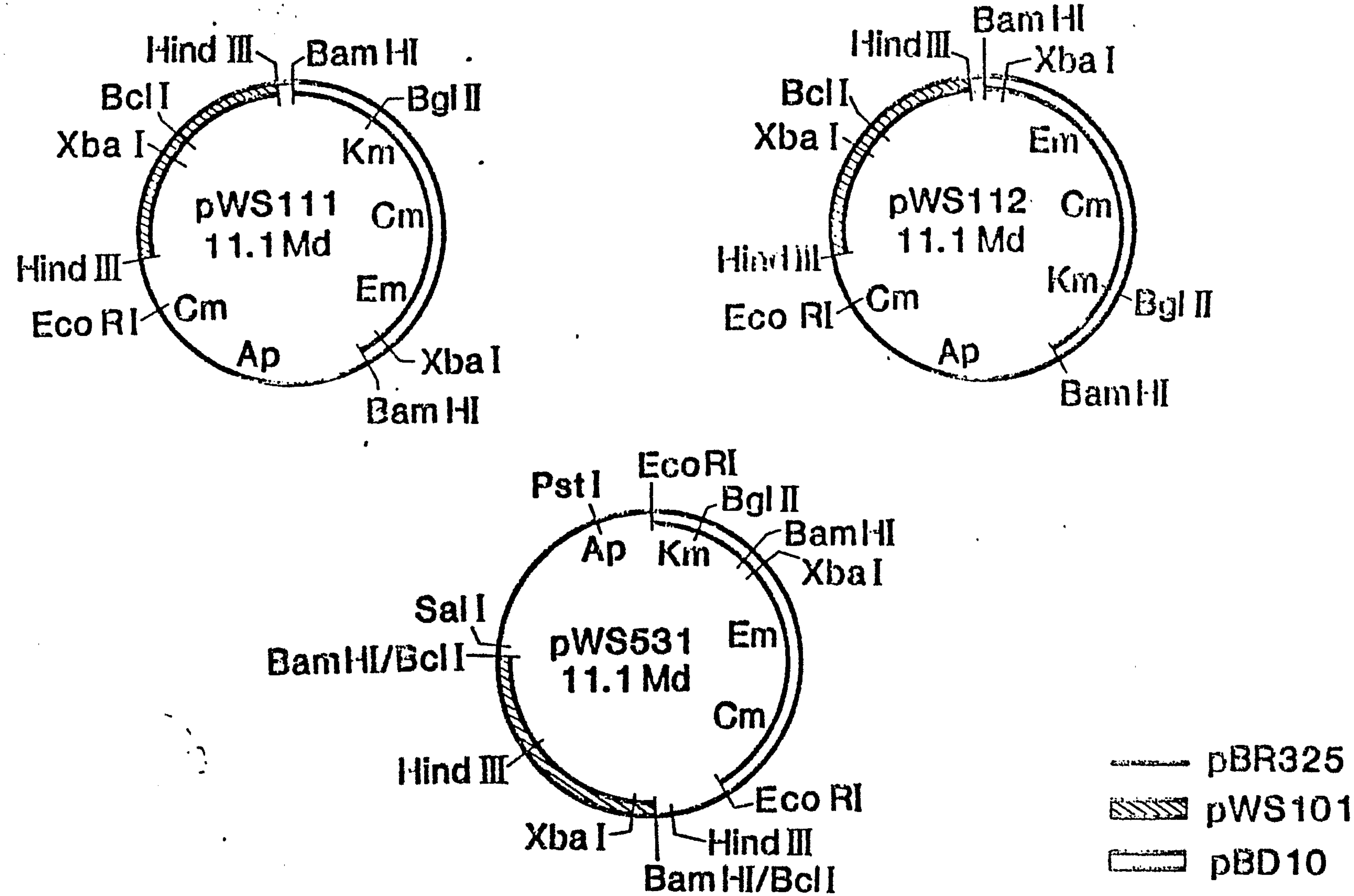

Fig. 4 Potential E. coli/Coryneform shuttle vectors constructed between pBR322 and pHY416